# EUROPÄISCHE PATENTSCHRIFT

(11) **EP 1 257 247 B1**
(45) Veröffentlichungstag und Bekanntmachung des Hinweises auf die Patenterteilung: **11.10.2006**
(21) Anmeldenummer: 01919287.1
(22) Anmeldetag: 16.02.2001
(51) Int. Cl.: A61K 8/02, A61Q 11/00, A61P 1/02

(54) **TREIBGASHALTIGES ZAHNREINIGUNGSMITTEL**
PROPELLANT-CONTAINING TOOTH CLEANING AGENT
PRODUITS D'HYGIENE DENTAIRE CONTENANT DES GAZ PROPULSEURS

(30) Priorität: 25.02.2000 DE 10008836
(43) Veröffentlichungstag der Anmeldung: 20.11.2002
(73) Patentinhaber: Henkel Kommanditgesellschaft auf Aktien, 40589 Düsseldorf (DE)
(72) Erfinder: LEINEN, Hans, Theo, 40229 Düsseldorf (DE); POSCHEN, Guido, 79599 Wittlingen (DE); WÜLKNITZ, Peter, 42799 Leichlingen (DE); BRESSLER, Christian, 60385 Frankfurt (DE)
(86) Internationale Anmeldenummer: PCT/EP2001/001723
(87) Internationale Veröffentlichungsnummer: WO 2001/062211

(56) Entgegenhaltungen:
- EP-A- 0 180 483
- WO-A-94/02109
- AU-A- 4 282 485
- DE-A- 1 419 184
- DE-A- 2 001 317
- US-A- 3 639 568
- DATABASE WPI Week 198033 Derwent Publications Ltd., London, GB; AN 1980-57547C XP002167868 & JP 55 085513 A (LION DENTIFRICE)

## Beschreibung

Die Erfindung betrifft ein treibgashaltiges Zahnreinigungs- und pflegemittel in einem Spendersystem, welches bei Betätigung eines Ventils als Pasten- oder Gelstrang abgegeben wird und zeitverzögert durch Freisetzung des Treibgases langsam aufquillt und einen feinblasigen Schaum entwickelt, der über lange Zeit hinweg stabil bleibt.

Zahnremigungsmittel sind in verschiedenen Formen auf dem Markt und dienen in erster Linie der Reinigung der Zahnoberfläche. Sie enthalten üblicherweise eine Kombination aus Poliermitteln, Feuchthaltemitteln, Tensiden, Bindemitteln, Aromastoffen und fluoridhaltigen sowie antimikrobiellen Wirkstoffen. Neben Zahnpulvern, die wegen ihrer erhöhten Abrasivität eine untergeordnete Rolle spielen, werden Zahnreinigungsmittel vor allem in Pasten-, Creme-und transluzenter oder transparenter Gelform angeboten. In den letzten Jahren haben auch Liquid- oder Flüssigzahncremes und Mundwässer zunehmend an Bedeutung gewonnen. Ein Nachteil der Produkte in Pasten-, Creme- oder Gelform ist deren vergleichsweise hohe Viskosität und dadurch bedingte schlechte Verteilbarkeit und langsame Freisetzung der Wirk- und Aromastoffe.

Eine verbesserte Verteilbarkeit erreicht man durch aufgeschäumte Zahncremes, die üblicherweise aus Aerosolspendern abgegeben werden. Derartige Formulierungen sind aus der WO 99/22704, WO 82/03975, EP 0 208 009, AU 8542824, DE 2 217 953, DE 36 23 934, JP 5714520 A und der JP 5714521 A bekannt. Als Treibgase werden FCKW's, Kohlendioxid, Sauerstoff und niedrigsiedende Kohlenwasserstoffe eingesetzt. Fluor-Chlor-Kohlenwasserstoffen (FCKWs) sind wegen ihrer ökologischen Bedenklichkeit als Treibgase jedoch in vielen Ländern nicht mehr erlaubt. Generell handelt es sich bei Zahnpastaschäumen um Emulsionen oder Dispersionen, die aus den Spendern in bereits aufgeschäumter Form abgegeben werden. Dies hat den Nachteil, daß die Wirkstoffmenge oft viel zu niedrig ist. Derartige Zahnpastaschänme sind meist nur über kurze Zeit stabil. Darüber hinaus sind reine Kohlenwasserstofftreibgase mit den üblichen Zahnpastaformulierungen meist nicht kompatibel, da sie in diesen unlöslich sind, so daß es zu einer Phasentrennung kommt.

Aufgabe war es daher, ein Zahnreinigungs- und pflegemittel mit verbesserter Verteilbarkeit sowie effizienter Wirkstoff- und Aromafreisetzung zu entwickeln, das zunächst als nicht oder nur gering aufgeschäumter, gelartiger Strang aus einem Spender abgegeben wird und dann zeitverzögert, insbesondere erst bei Applikation im Mund, einen mousseartigen, "prickelnden" Schaum mit völlig neuartiger Sensorik ausbildet. Eine weitere Aufgabe bestand darin, die Zusammensetzung so zu optimieren, daß Treibgas und Zahnpastagrundlage eine stabile Dispersion ausbilden, die sich nicht trennt und das Leitungssystem des Spenders blockiert.

Diese Aufgabe wurde erfindungsgemäß gelöst durch ein Zahnreinigungsmittel enthaltend
a) wenigstens ein Poliermittel
b) wenigstens ein Feuchthaltemittel
c) wenigstens ein Tensid
d) wenigstens eine wasserunlösliche, nicht-derivatisierte Cellulose dadurch gekennzeichnet, daß es mit einem Treibgas oder Treibgasgemisch in einem Spendersystem mit manuell betätigbarem Ventil abgefüllt ist.

Durch die Kombination der Komponenten a) - c) mit einer wasserunlöslichen, nicht-derivatisierten Cellulose wird eine stabile Dispersion des Treibgas/Treibgasgemisches in den erfindungsgemäßen Zusammensetzungen ermöglicht. Auch bei längerer Lagerung sind die erfindungsgemäßen Zusammensetzungen gegen eine Separation des Treibgases/Treibgasgemisches stabil. Eine Blockierung des Leitungssystems des Spenders tritt nicht ein. Die erfindungsgemäßen Zusammensetzungen bilden durch den Zusatz der Cellulose besonders stabile Schäume, die nicht zerfließen. Der Anteil des Wassers an der erfindungsgemäßen Zusammensetzung beträgt 1 - 60 Gew.-%, vorzugsweise 5 - 50 Gew.-% und insbesondere 30 - 50 Gew.-%. Das Zahnreinigungsmittel weist üblicherweise einen pH-Wert von 5,5 - 9, vorzugsweise 6,5 - 8,0 und insbesondere 7,0 -7,3 auf.

Die erfindungsgemäßen Zusammensetzungen sind zur Herstellung eines Zahnrenigungsmittels für die Reinigung und Pflege der Zähne und zur Prävention von Zahn- und Zahnfleischerkrankungen geeignet. Vorzugsweise weist das erfindungsgemäße Mittel ohne Treibgas bei 25° C eine Viskosität von wenigstens 10000 mPa·s auf (Brookfield Rotationsviskosimeter RVF, Spindel 3 oder 4 bei 4rpm), welche einerseits ein Abfüllen der Zusammensetzung über die Ventilkopföffnung des Spenders ermöglicht und andererseits die Formbeständigkeit nach Entnahme des Mittels aus dem Spender gewährleistet, also ein Zerfließen verhindert. Das Mittel kann direkt, z. B. mit einer Zahnbürste appliziert werden, eignet sich jedoch bei Verdünnung mit Wasser auch besonders gut als Mundspülung, da der feine Schaum sehr leicht in Wasser dispergierbar ist. Damit die erfindungsgemäßen Zusammensetzungen zusammen mit dem Treibgas unter Verwendung handelsüblicher Abfüllanlagen durch die Ventilkopföffnung des Spenders abgefüllt werden können, ist es vorteilhaft, daß die Viskosität des Mittels ohne Treibgas bei 25° C nicht höher als 100000 mPa·s ist, wobei ein Bereich von 10000 - 80000 mPa·s und insbesondere zwischen 10000 und 50000 mPa·s bevorzugt ist (Brookfield Rotationsviskosimeter RVF, Spindel 3 - 5, 4rpm).

Ein weiterer Gegenstand der Erfindung ist ein Verfahren zur Herstellung eines Mittels für die Zahnpflege und -reinigung, dadurch gekennzeichnet, daß man aus einem Spendersystem durch manuelle Betätigung eines Ventils einen nicht oder nur wenig aufgeschäumten Zahnpastastrang auf einen zur mechanischen Reinigung der Zähne geeigneten Gegenstand aufbringt, wobei der Zahnpastastrang bei 20 °C und Normaldruck eine Expansionsrate von nicht mehr als 100 Vol.-% in 5 Sekunden aufweist und dann in einer Zeitspanne von 10 - 100 Sekunden auf nicht mehr als das 6-fache seines Volumens expandiert, und man das gashaltige Zahnpasta-Mousse zur Reinigung der Zahnoberflächen, Zahnzwischenräume und der Mundhöhle verwendet.

### 1. Treibgas

Vorzugweise ist das Treibgas oder Treibgasgemisch in den erfindungsgemäßen Zusammensetzungen in einer Menge von wenigstens 1 Gew.-% enthalten. Ebenso bevorzugt ist es, den Gehalt an Treibgas nicht höher als 10 Gew.-% bezogen auf die Gesamtzusammensetzung zu wählen, da das Mittel ansonsten einen zu voluminösen Schaum bildet. Besonders bevorzugt ist es, 3 - 4 Gew.-% des Treibgases/Treibgasgemisches einzusetzen. Die erfindungsgemäße Zusammensetzung soll aus dem Spender in Form eines nicht oder nur wenig aufgeschäumten gelartigen Stranges abgegeben werden, der bei Entweichen des Treibgas/Treibgasgemisches - vorzugsweise im Mundinnenraum - ein feinblasiges Mousse ausbildet. Dies gewährleistet eine höhere Wirkstoffmenge im Vergleich zu Präparaten, die den Spendern direkt als voluminöser Schaum entnommen werden, und eine im Vergleich zu den üblichen Zahnreinigungsmitteln in Creme-, Gel- oder Pastenform verbesserte Verteilbarkeit der Wirkstoffe sowie eine optimierte Freisetzung/Entfaltung der Aromastoffe. Dies hat u.a. ein besonders intensives, lange anhaltendes Frischgefühl im Mundraum zur Folge. Da ein Teil des Treibgases vorzugsweise erst bei der Applikation des Mittels im Mundinnenraum freigesetzt wird, sollten die verwendeten Treibgase physiologisch unbedenklich sein. Vorzugsweise enthält das Treibgas wenigstens eine Komponente, die bei Normaldruck (1 atm) einen Siedepunkt im Bereich von -5 - 35 °C, vorzugsweise 15 - 35 °C und insbesondere 20 - 30 °C hat. Kohlenwasserstoff-Treibgase sind aufgrund ihrer physiologischen Vertäglichkeit besonders vorteilhaft. In einer bevorzugten Ausführungsform enthält das Treibgas ein C₅-Alkan, vorzugsweise in einer Menge von wenigstens 10 Gew.-% bezogen auf die Gesamtmenge des Treibgases. Unter diesen - n-Pentan, 2-Methylbutan (= Isopentan) und Neopentan - ist das 2-Methylbutan mit einem Siedepunkt von 29 °C bevorzugt geeignet Vorzugsweise wird als Treibgas ein Gemisch eines C₄-Alkans und eines C₅-Alkans eingesetzt, wobei ein 1:3 - 3:1-Gemische (Gewichtsverhältnis) aus Isopentan/Isobutan erfindungsgemäß besonders gut geeignet sind, um die zeitverzögerte Aufschäumung des Zahnreinigungsmittels ("post-foaming" Effekt) zu gewährleisten. Derartige Formulierungen sind direkt nach der Abgabe aus dem Spender noch gelartig und quellen dann auf der Zahnbürste allmählich zu einem sehr stabilen Schaum auf, der sich besonders bei Körpertemperatur, also bei Applikation der Zusammensetzung im Mundinnenraum, relativ schnell entfaltet. Die zeitverzögerte Gasfreisetzung im Mundinnenraum bewirkt neben einer völlig neuen Sensorik vor allem eine verbesserte Aromaentfaltung mit einem intensivierten Frischegefühl.

### 2. Spender

Die Zahnreinigungsmittel werden in handelsüblichen Abfüllanlagen zusammen mit dem unter Druck verflüssigten Treibgas oder Treibgasgemisch in Spendersysteme abgefüllt, die ausreichend druckresistent sind, daß das Treibgas in der Zusammensetzung verflüssigt vorliegt. Als Spender für die Zusammensetzung eignet sich beispielsweise ein Behälter mit Ventil aus einem starren Material, der mit einer Vorrichtung ausgestattet ist, die zum Ausstoßen der in ihr bevorrateten Zusammensetzung (also Zahnreinigungsmittel und Treibgas) die Kontraktionskraft eines gedehnten Gummischlauches und/oder eines gedehnten Produktbehälters einsetzt. Ein solcher Spender ist beispielsweise in EP 69699 beschrieben. Auch die von der 3D Dispenser-Distributions GmbH unter der Bezeichnung FlexPack® angebotenen Behälter benutzen die Rückstellkraft eines gedehnten Gummis zum Ausstoßen der Zusammensetzung. Erfindungsgemäß bevorzugt geeignet sind Behälter, die einen gefalteten, im wesentlichen gasundurchlässigen flexiblen Innenbeutel aus einem chemisch inerten Kunststoff (z. B. PET) enthalten, der von einem elastischen, dickwandigen Gummischlauch umgeben ist. Derartige Spender sind beispielsweise in US 5,927,551, US 4,964,540 und EP 69738 beschrieben und werden von der Firma Exxel Container unter der Bezeichnung Atmos™ Dispensing System vertrieben. In Kombination mit derartigen Spendern kann auch reines Butan als Treibgas bereits einen "post-foaming" Effekt erzeugen.

Ebenso sind Zweikammer-Aerosoldosen mit Innenbeutel bevorzugt als Spender geeignet. Diese enthalten im Innenbeutel das Füllgut (Zahnreinigungsmittel mit verflüssigtem Gas) und in der äußeren Kammer ein weiteres Treibgas, das zum Ausstoßen der Zusammensetzung bei Betätigung des Ventils dient. Derartige Systeme können entweder durch eine Öffnung am Dosenboden oder - wie beim sogenannten "under cup system" - durch Anheben des Ventils befüllt werden.

### 3. Cellulose

Als Konsistenzregulator ist wasserunlösliche, nicht-derivatisierte Cellulose ein essentieller Bestandteil der erfindungsgemäßen Zusammensetzung, der die Bildung eines besonders stabilen Schaums gewährleistet. In Kombination mit Tensiden und Lösungsvermittlern ermöglicht sie eine stabile Dispersion des Treibgases in der Zahnpasta-Grundmasse. In der erfindungsgemäßen Zusammensetzung trägt sie auch dazu bei, eine Separation der flüssigen und festen Bestandteile zu verhindern. Die Cellulose wird üblicherweise in einer Menge von 0,01 - 5,0 Gew.-% Aktivsubstanz, vorzugsweise 0,01- 2,0 Gew.-% bezogen auf die Gesamtzusammensetzung eingesetzt.

Im Sinne der Erfindung wird unter wasserunlöslich eine Löslichkeit von weniger als 1 Gew.-% in Wasser bei 20 °C verstanden, d. h. daß in 100 g einer gesättigten wässrigen Lösung bei 20 °C weniger als 1 Gew.-% der Cellulose gelöst ist. Im Sinne der Erfindung werden Cellulosen als nicht-derivatisiert bezeichnet, deren Hydroxyl-Wasserstoff-Atome nicht durch andere chemische Gruppen substituiert sind.

Cellulose ist eine ubiquitäre pflanzliche Gerüstsubstanz. Sie kann in nativer oder gereinigter Form eingesetzt werden. Gereinigte Cellulose ist aufgrund ihrer guten physiologischen Verträglichkeit für den Einsatz in der Oralhygiene bevorzugt geeignet Das Molekulargewicht der Cellulose liegt üblicherweise unter 800000, vorzugsweise bei 10000 - 700000 und insbesondere bei 20000 - 500000.

Bevorzugt geeignet sind mikrokristalline oder pulverförmige Cellulosen, die beispielsweise von J. Rettenmaier & Söhne unter der Bezeichnung Arbocel® und Vitacel® angeboten werden. Cellulose mit einer durchschnittlichen Primärpartikelgröße von weniger als 1 µm, insbesondere Arbocel® CGP 5000, eine hochviskose Paste aus Pulvercellulose mit thixotropen Eigenschaften, ist besonders bevorzugt geeignet. Arbocel®-Cellulosen sind äußerst effektive Verdicker, die auch bei sehr niedriger Einsatzkonzentration die Viskosität der Zusammensetzungen beträchtlich erhöhen, gegen ionische Bestandteile inert sind und sich gut mit weiteren Verdickungsmitteln kombinieren lassen. Durch Kombination mit Cellulosen vom Arbocel®-Typ erhält man Formulierungen, die einerseits dünnflüssig genug sind, um eine Abfüllung über die Ventilkopföffnung des Spendersystems zu ermöglichen und andererseits nach Entnahme aus dem Spendersystem einen stabilen Schaum mit Volumen entwickeln, der ein völlig neuartiges Mundgefühl bewirkt. Der Schaum, der sich aus dem Gelstrang entwickelt, ist über mehrere Stunden hinweg stabil.

### 4. Poliermittel

Als Poliermittel, Putzkörper oder Abrasivmittel werden in der Regel wasserunlösliche anorganische Stoffe eingesetzt, die den Zahnbelag mechanisch entfernen, ohne den Zahnschmelz oder das Dentin zu schädigen. Besonders vorteilhaft kann die Verwendung sehr feinteiliger Poliermittel mit einer mittleren Korngröße von 1 - 200 µm, vorzugsweise 1 - 50 µm und insbesondere 1-10 µm sein. Das Poliermittel wird in den erfindungsgemäßen Zusammensetzungen in einer Menge von 5 - 50 Gew.-%, vorzugsweise 8 - 30 Gew.-% und insbesondere 10 - 20 Gew.-% bezogen auf die Gesamtzusammensetzung eingesetzt. Häufig ist es besonders vorteilhaft, eine Kombination von Poliermitteln zu verwenden.

Erfindungsgemäß geeignet sind Kieselsäuren, wasserunlösliche Metaphosphate wie z. B. Natriummetaphosphat, Calciumphosphate wie z. B. Tricalciumphosphat, Calciumhydrogenphosphat, Calciumhydrogenphosphat-Dihydrat und Calciumpyrophosphat, Calciumcarbonat (Kreide), Magnesiumcarbonat, Magnesiumdihydrogenphosphat, Trimagnesiumphosphat, Aluminiumoxid, calciniertes Aluminiumoxid, Aluminiumhydroxid und Aluminiumoxid-Hydrate, Hydroxylapatit sowie verschiedene Silikate. Auch Natriumhydrogencarbonat ist als Poliermittel erfindungsgemäß einsetzbar, insbesondere in Mischung mit anderen Poliermitteln.

Vorzugsweise enthält die Zusammensetzung als Poliermittel wenigstens eine Verbindung aus der Gruppe der Kieselsäuren oder Silikate. Unter den Kieselsäure-Poliermitteln sind Fällungs- und Gelkieselsäuren bevorzugt geeignet, da sie bei der Herstellung in ihren Eigenschaften breit variiert werden können und besonders gut mit Fluorid-Wirkstoffen verträglich sind. Sie eignen sich auch besonders gut für die Herstellung von Gel- oder Liquid-Zahncremes.

Gelkieselsäuren werden durch Umsetzung von Natriumsilikatlösungen mit starken wäßrigen Mineralsäuren unter Ausbildung eines Hydrosols, Alterung zum Hydrogel, Waschen und anschließendem Trocknen erzeugt. Erfolgt die Trocknung unter schonenden Bedingungen auf Wassergehalte von 15-35 Gew.-%, so werden sogenannte Hydrogelkieselsäuren erhalten, wie sie beispielsweise aus US 4,153,680 bekannt sind. Durch Trocknung auf Wassergehalte unterhalb von 15 Gew.-% erfolgt eine irreversible Schrumpfung der vorher lockeren Struktur des Hydrogels zur dichten Struktur des sogenannten Xerogels. Solche Xerogelkieselsäuren sind z. B. in US 3,538,230 beschrieben.

Fällungskieselsäuren werden aus wässrigen Alkalisilikat-Lösungen durch Ausfällung mit Mineralsäuren hergestellt und zwar unter Bedingungen, bei denen eine Aggregation zum Sol und Gel nicht eintreten kann. Geeignete Verfahren zur Herstellung von Fällungskieselsäuren sind z. B. in DE-OS 25 22 486 und in DE-OS 31 14 493 beschrieben.

Erfindungsgemäß geeignete Kieselsäurepoliermittel sind beispielsweise unter der Bezeichnung Sident® 8, Zeodent® 113 und 623 , Sorbosil® AC39, Tixosil® 123 und 73 im Handel.

Unter den Silikaten sind verschiedene Aluminiumsilikate und Zirkoniumsilikate als Poliermittel bekannt. Auch Natrium-Aluminiumsilikate können als Poliermittel geeignet sein, wie z. B. synthetische Zeolithe, insbesondere Zeolith A.

Ferner können auch teilchenförmige organische Polymere, z. B. Polymethacrylat, Polymethacrylat oder Polyethylen und Polypropylen einer mittleren Teilchengröße von ca. 5 - 15 µm und einem durchschnittlichen Molekulargewicht von 3000 g/mol als Putzkörper eingesetzt werden.

### 5. Feuchthaltemittel

Feuchthaltemittel werden Zahncremes nicht nur zugesetzt, um sie vor Austrockung zu schützen, sie dienen auch der Konsistenzgebung und der Kältestabiltät. Als Feuchthaltemittel können toxikologisch unbedenkliche Polyole eingesetzt werden, wie z. B. Sorbitol, Xylitol, Glycerin, Mannitol, 1,2-Propylenglycol oder Gemische dieser Polyole. Auch Polyethylenglycole mit Molekulargewichten von 400 - 2000 können anteilig als Feuchthaltemittelkomponenten enthalten sein. In einer bevorzugten Ausführungsform enthält das erfindungsgemäße Mittel als Feuchthaltemittel wenigstens Sorbitol, Glycerin oder Xylitol bzw. ein beliebiges Gemisch dieser Substanzen. Das Feuchthaltemittel oder das Gemisch aus Feuchthaltemitteln ist in der Gesamtzusammensetzung in einer Menge von
15 - 85 Gew.-%, vorzugsweise 20 - 70 Gew.-% und insbesondere 30 - 50 Gew.-% enthalten. Vorzugsweise enthält die erfindungsgemäße Zusammensetzung ein Gemisch aus Feuchthaltemitteln, insbesondere mit einem Anteil an Sorbit/Polyethylenglycol im Gewichtsverhälnis 10: (0,1-1).

### 6. Oberflächenaktive Substanzen

Eine weitere Verbesserung der Reinigungswirkung der erfindungsgemäßen Zahnreinigungsmittel wird durch Zusatz eines geeigneten oberflächenaktiven Tensids oder Tensidgemisches erzielt. Eine ausführliche Auflistung geeigneter Tenside ist in U.S. 3,988,433 enthalten, die Teil der Offenbarung der vorliegenden Anmeldung sein sollen. Der Zusatz von Tensiden dient außerdem zur Erzeugung eines Schaums beim Zähnebürsten und damit zur besseren Wirkstoffverteilung, zur Stabilisierung der treibgashaltigen Dispersion sowie zur Emulgierung oder Solubilisierung der ggf. enthaltenen Fette, Öle, Wachse und der Aromaöle. Das Tensid oder Tensidgemisch wird in den erfindungsgemäßen Zusammensetzungen in einer Menge von 0,1 - 10 Gew.-%, vorzugweise 0,3 - 7 und insbesondere 1- 5 Gew.-% bezogen auf die Gesamtzusammensetzung eingesetzt. Vorzugsweise enthält die Zusammensetzung wenigstens ein Tensid aus der Gruppe der Aniontenside.

### Aniontenside

Geeignete Tenside mit guter Schaumwirkung sind anionische Tenside, die auch eine gewisse enzymhemmende Wirkung auf den bakteriellen Stoffwechsel des Zahnbelags haben. Hierzu gehören Alkali- oder Ammoniumsalze - insbesondere Natriumsalze von C₈-C₁₈-Alkancarbonsäuren, von Alkylpolyglycolethersulfaten mit 12 - 16 C-Atomen in der linearen Alkylgruppe und 2 - 6 Glycolethergruppen im Molekül, von linearen Alkan-(C₁₂-C₁₈)-sulfonaten, Sulfobernsteinsäuremonoalkyl-(C₁₂-C₁₈)-estern, sulfatierten Fettsäuremonoglyceriden, sulfatierten Fettsäurealkanolamiden, Sulfoessigsäurealkyl-(C₁₂-C₁₆)-estern, Acylsarcosinen, Acyltauriden und Acylisethionaten mit jeweils 8 - 18 C-Atomen in der Acylgruppe. Bevorzugt ist die Verwendung wenigstens eines Aniontensids, insbesondere eines Natriumalkylsulfats mit 12 - 18 C-Atomen in der Alkylgruppe. Ein derartiges Tensid ist Natriumlaurylsulfat, das beispielsweise unter der Bezeichnung Texapon® K1296 im Handel ist.

### Zwitterionische und ampholytische Tenside

Auch zwitterionische und ampholytische Tenside können, bevorzugt in Kombination mit anionischen Tensiden, eingesetzt werden. Zu den erfindungsgemäß einsetzbaren Amphotensiden gehört z. B. Alkylaminopropancarbonsäure. Die bekannteste und am weitesten verbreitete Gruppe der zwitterionischen Tenside ist die der Betain-Tenside, wie z. B. Alkyldimethylcarboxymethylbetain und Acylaminoalkyldimethylcarboxymethylbetain. Bevorzugt geeignet sind Kokosacylaminopropyldimethylammoniumglycinate, die unter der INCI-Bezeichnung Cocamidopropylbetain bekannt sind. Solche Produkte sind beispielsweise unter der Bezeichnung Tego-Betain® BL 215 und ZF 50 sowie Genagen® CAB im Handel.

### Niotenside

Besonders geeignet zur Unterstützung der Reinigungswirkung sind nichtionogene Tenside, unter denen die Anlagerungsprodukte von Ethylenoxid an Fettalkohole, an Fettsäuren, an Fettsäuremonoglyceride, an Sorbitan-Fettsäuremonoester oder an Methylglucosid-Fettsäuremonoester bevorzugt sind. Die angelagerte Menge an Ethylenoxid sollte dabei so hoch sein, daß die Tenside wasserlöslich sind, d. h. es sollte wenigstens 1 g des Produktes in 11 Wasser bei 20 °C klar löslich sein. Erfindungsgemäß bevorzugt als nichtionogenes Tensid sind ethoxylierte Glycerylmonoalkylester mit einem Ethoxylierungsgrad von 20 - 60 und einer C₈-C₁₈-Alkylkette, wie beispielsweise PEG-30-Glycerylstearat, das z. B. unter der Bezeichnung Tagat® S im Handel ist, und auch als Lösungsvermittler fungiert.

Als nichtionogene Tenside eignen sich aber auch Alkyl-(oligo)-glycoside mit 8 - 16 C-Atomen in der Alkylgruppe und einem Oligomerisationsgrad des Glycosidrestes von 1 - 4 Alkyl-(oligo)-glycoside, ihre Herstellung und Verwendung als oberflächenaktive Stoffe sind z. B. aus US-A-3,839,318, DE-A-20 36 472, EP-A-77 167 oder WO-A-93/10132 bekannt. Bezüglich des Glycosidrestes gilt, daß sowohl Monoglycoside (x = 1), bei denen ein Monosaccharidrest glycosidisch an einen Fettalkohol mit 10 bis 16 C-Atomen gebunden ist, als auch oligomere Glycoside mit einem Oligomerisationsgrad x bis 10 geeignet sind. Der Oligomerisationsgrad ist dabei ein statistischer Mittelwert, dem eine für solche technischen Produkte übliche Homologenverteilung zugrunde liegt. Bevorzugt eignet sich als Alkyl-(oligo)-glycosid ein Alkyl-(oligo)-glucosid der Formel RO(C₆H₁₀O)ₓ-H, in der R eine Alkylgruppe mit 12 bis 14 C-Atomen ist und x einen Mittelwert von 1 bis 4 hat. Auch Aminoxide sind als Tenside geeignet.

Um eine intensive Reinigungswirkung in Kombination mit guter Schaumbildung und eine ausreichende Solubilisierung der Treibgase zu erreichen, wird in den erfindungsgemäßen Zusammensetzungen vorzugsweise ein Gemisch aus Anion- und Niotensid oder ein Gemisch aus Aniontensid, Niotensid und Betain verwendet, beispielsweise Natriumlaurylsulfat/PEG-30-Glycerylstearat/Cocamidopropylbetain.

### Lösungsvermittler

Zur besseren Solubilisierung der Treibgase und der ggf. in den erfindungsgemäßen Zusammensetzungen enthaltenen Öl- und Wachskomponenten dienen neben Ethanol insbesondere nichtionogene Lösungsvermittler aus der Gruppe der oberflächenaktiven Substanzen. Hierzu zählen, wie bereits erwähnt, insbesondere ethoxylierte Verbindungen mit einem Ethoyxlierungsgrad von 20 - 60. Besonders geeignet für diesen Zweck sind ethoxylierte Fettsäureglyceride, ethoxylierte Fettsäuresorbitanpartialester oder Fettsäurepartialester von Glycerin- oder Sorbitan-Ethoxylaten. Lösungsvermittler aus der Gruppe der ethoxylierten Fettsäureglyceride umfassen vor allem Anlagerungsprodukte von 20 bis 60 Mol Ethylenoxid an Mono- und Diglyceride von linearen Fettsäuren mit 12 bis 18 C-Atomen oder an Triglyceride von Hydroxyfettsäuren wie Oxystearinsäure oder Ricinolsäure. Weitere geeignete Lösungsvermittler sind ethoxylierte Fettsäuresorbitanpartialester, z. B. Anlagerungsprodukte von 20 bis 60 Mol Ethylenoxid an Sorbitanmonoester und Sorbitandiester von Fettsäuren mit 12 bis 18 C-Atomen. Ebenfalls geeignete Lösungsvermittler sind Fettsäurepartialester von Glycerin- oder Sorbitan-Ethoxylaten; das sind bevorzugt Mono- und Diester von C₁₂-C₁₈-Fettsäuren und Anlagerungsprodukten von 20 bis 60 Mol Ethylenoxid an 1 Mol Glycerin oder an 1 Mol Sorbit.

### Kationische Tenside

Ferner sind auch einige kationische Tenside wie z. B. Alkyl-trimethylammoniumchlorid, Alkyldimethylbenzylammoniumchlorid, Alkylpyridiniumchlorid, Alkyldimethylhydroxyethyl-ammoniumchlorid, Acylimidazoliniummethosulfate und Acyloxyethyltrimethylammonium-chlorid als Tenside in den erfindungsgemäßen Mitteln geeignet.

### 7. Öl/Fett- und Wachskomponenten

In einer weiteren bevorzugten Ausführungsform enthält die erfindungsgemäße Zusammensetzung auch Öl/Fett- und/oder Wachskomponenten. In Kombination mit den Tensiden ermöglichen sie eine partielle Solubilisierung des Treibgases oder Treibgasgemisches und verleihen der Zahnpastagrundmasse Glanz und Geschmeidigkeit. Erfindungsgemäß können natürliche, chemisch modifizierte und synthetische Wachse, Fette und Öle alleine oder in beliebiger Kombination eingesetzt werden. Die Öl/Fett- und Wachskomponenten sind in einer Menge von 0,1 bis 10 Gew.-%, bezogen auf die Gesamtzusammensetzung, vorzugsweise 0,5 bis 5 Gew.-% und insbesondere 0,8 - 3 Gew.-% enthalten.

### Öle/Fette

Im Sinne der Erfindung werden hierunter Di- und Triglyceride mit flüssiger bis fester Konsistenz sowie Kohlenwasserstoffe und Silikonöle verstanden, die natürlichen oder synthetischen Ursprungs sein können. Erfindungsgemäß kann es vorteilhaft sein als Ölkomponenten Triglyceride und/oder ein Paraffinöl einzusetzen Diese können pflanzlichen, tierischen oder synthetischen Ursprungs sein.

Di- und Triglyceride sind die Di- und Triester von Fettsäuren mit Glycerin, also Acylglycerine, wobei das Glycerin mit gleichen oder mit unterschiedlichen Fettsäuren oder Fettsäurederivaten (z. B. Lecithin) verestert sein kann. Bei den Fettsäuren handelt es sich vorzugsweise um C₆-C₃₀-Fettsäuren, die gesättigt oder ungesättigt sowie verzweigt oder unverzweigt sein können. Hierzu gehören u. a. die Produkte der Veresterung von Glycerin mit natürlich vorkommenden Fettsäuren wie beispielsweise Capronsäure, Önanthsäure, Caprylsäure, Pelargonsäure, Caprinsäure, Undecansäure, Laurinsäure, Tridecansäure, Myristinsäure, Pentadecansäure, Palmitinsäure, Margarinsäure, Stearinsäure, Nonadecansäure, Arachinsäure, Behensäure, Lignocerinsäure, Cerotinsäure, Melissinsäure, 2-Ethylhexansäure, Isotridecansäure, Isostearinsäure, Palmitoleinsäure, Ölsäure, Elaidinsäure, Petroselinsäure, Elaeostearinsäure, Erucasäure, Linolsäure, Linolensäure, Arachidonsäure, Clupanodonsäure, Docosahexaensäure und Gadoleinsäure sowie deren technische Mischungen, die z. B. bei der Druckspaltung von natürlichen Fetten und Ölen oder der Dimerisierung von ungesättigten Fettsäuren anfallen. Besonders geeignet kann die Verwendung natürlicher Fette und Öle wie z. B. Rindertalg, Erdnußöl, Rüböl, Baumwollsaatöl, Sojaöl, Sonnenblumenöl, Palmöl, Palmkernöl, Leinöl, Mandelöl, Rizinusöl, Maisöl, Olivenöl, Rapsöl, Sesamöl, Kakaobutter und Kokosfett und dergleichen sein. Auch die hydrierten oder gehärteten Öle, z. B. hydriertes Sojaöl, Rizinusöl und Erdnußöl können verwendet werden.

Erfindungsgemäß einsetzbar sind auch dünn- bis dickflüssige Silikonöle oder natürliche und synthetische Kohlenwasserstoffe wie beispielsweise dünn- bis dickflüssige Paraffinöle, Isohexadecan, Isoeicosan oder Polydecene, die z. B. unter der Bezeichnung Emery® 3004, 3006, 3010, Ethylflo® oder Nexbase® 2004G erhältlich sind. Auch der Einsatz sogenannter inverser Fette (z. B. Ester vicinaler Tricarbonsäuren mit C₆-C₃₀-Fettalkoholen) oder von Glyceryltrialkyethern ist hier denkbar.

### Wachse

Unter Wachsen werden natürlich oder künstlich gewonnene Stoffe mit folgenden Eigenschaften verstanden: sie sind von fester bis brüchig harter Konsistenz, grob bis feinkristallin, durchscheinend bis opak, jedoch nicht glasartig und schmelzen oberhalb von 35° C ohne Zersetzung. Sie sind schon wenig oberhalb des Schmelzpunktes niedrigviskos und nicht fadenziehend und zeigen eine stark temperaturabhängige Konsistenz und Löslichkeit. Die häufigsten Vertreter aus der Gruppe der Wachse sind chemisch gesehen Ester aus Fettsäuren und höheren Fettalkoholen, die tierischen und pflanzlichen Ursprungs sind. Erfindungsgemäß verwendbar sind natürliche pflanzliche Wachse, wie z. B. Jojobaöl; Candelillawachs, Carnaubawachs, Japanwachs, Espartograswachs, Korkwachs, Guarumawachs, Reiskeimölwachs, Zuckerrohrwachs, Ouricurywachs, Montanwachs, Sonnenblumenwachs, Fruchtwachse wie Orangenwachse, Zitronenwachse, Grapefruitwachs, Lorbeerwachs (=Bayberrywax) und tierische Wachse, wie z. B. Bienenwachs, Schellackwachs, Walrat, Wollwachs und Bürzelfett. Es kann auch vorteilhaft sein, hydrierte oder gehärtete Wachse einzusetzen. Zu den erfindungsgemäß verwendbaren natürlichen Wachsen zählen auch die Mineralwachse, wie z. B. Ceresin und Ozokerit oder die petrochemischen Wachse, wie Paraffinwachse (z. B. Vaseline) und Mikrowachse. Als Wachskomponente sind auch chemisch modifizierte Wachse, wie z. B. Montanesterwachse, Sasolwachse und hydrierte Jojobawachse einsetzbar. Zu den synthetischen Wachsen, die erfindungsgemäß einsetzbar sind, zählen beispielsweise auch wachsartige Polyalkylenwachse und Silikonwachse.

Erfindungsgemäß einsetzbar sind auch synthetische Wachse, insbesondere die Ester aus C₆-C₃₀-Fettsäuren mit C₆-C₃₀-Fettalkoholen, die natürlichen oder synthetischen Ursprungs sein können. Sowohl die Fettsäure-Komponente als auch die Fettalkohol-Komponente kann geradkettig oder verzweigt und gesättigt oder einfach oder mehrfach ungesättigt sein.

Erfindungsgemäß kann es vorteilhaft sein, die Wachskomponente aus der Gruppe der pflanzlichen oder der tierischen und/oder der Paraffinwachse bzw. einem beliebigen Gemisch dieser Wachse zu wählen.

### 8. Weitere Bindemittel/Verdickungsmittel

Das erfindungsgemäße Mittel enthält in einer bevorzugten Ausführungsform neben Cellulose zusätzlich wenigstens ein weiteres Binde- oder Verdickungsmittel, das in den erfindungsgemäßen Zusammensetzungen in einer Gesamtmenge von 0,1 - 5 Gew.-%, vorzugsweise 0,1 - 3 Gew.-% und insbesondere 0,5 - 2 Gew.-% enthalten ist. Verwendet werden beispielsweise natürliche und/oder synthetische wasserlösliche Polymere wie Alginate, Carragene, Agar-Agar, Guar-Gum, Gummi arabicum, Succinoglycan-Gum, Guarmehl, Johannisbrotkernmehl, Tragant, Karaya-Gummi, Xanthan, Pektine, derivatisierte Cellulosen wie z. B. Carboxymethylcellulose, Hydroxyethylcellulose oder Methylhydroxypropylcellulose, hydrophob modifizierte Cellulosen, Stärke- und Stärkeether. Auch wasserlösliche Carboxyvinylpolymere (z. B. Carbopol®-Typen), Polyvinylalkohol, Polyvinylpyrrolidon und höhermolekulare Polyethylenglycole (insbesondere solche mit Molekulargewichten von 10² - 10⁶ D) eignen sich als weitere Binde- und Verdickungsmittel. Ebenso können Schichtsilikate und feinteilige Kieselsäuren (Aerogelkieselsäuren und pyrogene Kieselsäuren) diese Funktion erfüllen.

### 9. Zusätzliche Wirkstoffe

Eine weitere bevorzugte Ausführungsform des Zahnreinigungsmittels ist dadurch gekennzeichnet, daß es als zusätzliche Wirkstoffe Antikarieswirkstoffe, antimikrobielle Wirkstoffe, Zahnstein-Inhibitoren, Remineralisierungswirkstoffe, Geschmacksstoffe oder eine beliebige Kombination dieser Stoffe enthält.

### Antikaries-Wirkstoffe

Zur Bekämpfung und Vorbeugung gegen Karies eignen sich vor allem Fluorverbindungen, bevorzugt aus der Gruppe der Fluoride oder Monofluorophosphate in einer Menge von 0,1 - 0,5 Gew.-% Fluor. Geeignete Fluorverbindungen sind z. B. Natriumfluorid, Kaliumfluorid, Zinnfluorid, Dinatriummonofluorophosphat (Na₂PO₃F), Dikalium-monofluorophosphat oder das Fluorid einer organischen Aminoverbindung.

### Antimikrobielle Wirkstoffe

Als antimikrobielle Komponente eignen sich z. B. Phenole, Resorcine, Bisphenole, Salicylanilide und -amide und deren halogenierte Derivate, halogenierte Carbanilide und p-Hydroxybenzoesäureester. Unter den antimikrobiellen Komponenten sind diejenigen besonders geeignet, die das Wachstum von Plaque-Bakterien hemmen. Beispielsweise sind halogenierte Diphenylether, wie 2,4-Dichlor-2'-hydroxydiphenylether, 4,4'-Dichlor-2'-hydroxydiphenylether, 2,4,4'-Tribrom-2'-hydroxydiphenylether, 2,4,4'-Trichlor-2'-hydroxydiphenylether (Triclosan) als antimikrobielle Wirkstoffe geeignet. Neben Bromchlorophen, Bisbiguaniden wie Chlorhexidin und Alexidin, Phenylsalicylsäureestern und 5-Amino-1,3-bis(2-ethylhexyl)-hexahydro-5-methylpyrimidin (Hexetidin) wirken auch Zink- und Kupferionen antimikrobiell, wobei synergistische Effekte insbesondere in Kombination mit Hexetidin und Triclosan auftreten. Auch quartäre Ammoniumverbindungen, wie z. B. Cetylpyridiniumchlorid, Benzalkoniumchlorid, Domiphenbromid und Dequaliniumchlorid sind einsetzbar. Als antimikrobiell wirksam haben sich auch Octapinol, Octenidine und Sanguinarin erwiesen. Die antimikrobiellen Wirkstoffe, die auch in Form von Nanopartikeln zugegeben werden können, werden bevorzugt in Mengen von 0,01-1 Gew.-% in den erfindungsgemäßen Mitteln eingesetzt. Besonders bevorzugt wird Irgacare® MP in einer Menge von 0,01- 0,3 Gew.-% verwendet.

### Zahnsteininhibitoren

Bei Zahnstein handelt es sich um Mineralablagerungen, die dem natürlichen Zahnschmelz sehr ähnlich sind. Um eine Zahnsteinbildung zu inhibieren, werden den erfindungsgemäßen Zahnreinigungsmitteln Stoffe zugesetzt, die gezielt in die Kristallkeimbildung eingreifen und bereits vorhandene Keime am Weiterwachsen hindern. Hierbei handelt es sich beispielsweise um kondensierte Phosphate, die bevorzugt gewählt werden aus der Gruppe der Tripolyphosphate, der Pyrophophate, der Trimetaphosphate oder deren Gemischen. Sie werden in Form ihrer Alkali- oder Ammoniumsalze, bevorzugt in Form ihrer Natrium- oder Kaliumsalze eingesetzt. Wäßrige Lösungen dieser Phosphate reagieren typischerweise alkalisch, so daß der pH-Wert der erfindungsgemäßen Zahnpflegemittel ggf. durch Zusatz von Säure auf Werte von 7,5 - 9 eingestellt wird. Als Säuren können dabei z. B. Zitronensäure, Phosphorsäure oder saure Salze, z. B. NaH₂PO₄ verwendet werden. Der gewünschte pH-Wert des Zahnpflegemittels kann aber auch durch Zusatz saurer Salze der kondensierten Phosphate, also z. B. K₂H₂P₂O₇, eingestellt werden.

Auch Gemische verschiedener kondensierter Phosphate und/oder hydratisierte Salze der kondensierten Phosphate sind erfindungsgemäß einsetzbar. Zahnsteininhibitoren werden üblicherweise in Mengen von 0,1 - 5 Gew.-%, bevorzugt 0,1 - 3 Gew.-% und insbesondere 0,1 - 2 Gew.-% in den erfindungsgemäßen Mitteln eingesetzt.

Weitere geeignete Zahnsteininhibitoren sind Organophosphonate wie 1-Azacycloheptan-2,2-diphosphonat (Na-Salz), 1-Hydroxyethan-1,1-diphosphonat (Na-Salz) und Zinkcitrat.

### Remineralsierungswirkstoffe

Die erfindungsgemäßen Mittel enthalten vorzugsweise auch Stoffe, die eine Remineralisierung des Zahnschmelzes fördern und Dentalläsionen zu schließen vermögen. Diese sind üblicherweise in einer Gesamtmenge von 0,1 - 10 Gew.-%, vorzugsweise 0,1 - 5 Gew.-% und insbesondere 0,1 - 3 Gew.-% enthalten. Hierzu gehören z. B. Fluoride, Phosphatsalze des Calciums wie z. B. Calciumglycerinphosphate, Calciumhydrogenphopsphat, Hydroxylapatit, Fluorapatit, F-dotierter Hydroxylapatit, Dicalciumphosphatdihydrat sowie Calciumfluorid. Aber auch Magnesiumsalze wie z. B. Magnesiumsulfat, Magnesiumfluorid oder Magnesiummonofluorophosphat wirken remineralisierend.

### Geschmacksstoffe

Vorzugsweise enthalten die erfindungsgemäßen Mittel Geschmacksstoffe, zu denen z. B. Süßungsmittel und/oder Aromaöle gehören. Als Süßungsmittel eignen sich beispielsweise Saccharinate (insbesondere Natriumsaccharinat), Cyclamate (insbesondere Natriumcyclamat) sowie Sucrose, Lactose, Maltose oder Fructose. Als Aromaöle kommen alle für Mund- und Zahnpflegemittel gebräuchlichen natürlichen und synthetischen Aromen in Frage. Natürliche Aromen können sowohl in Form der aus den Drogen isolierten etherischen Öle (Mischung) als auch in Form der hieraus isolierten Einzelkomponenten verwendet werden. Bevorzugt sollte wenigstens ein Aromaöl aus der Gruppe Pfefferminzöl, Krausenminzöl, Anisöl, Sternanisöl, Kümmelöl, Eukalyptusöl, Fenchelöl, Zimtöl, Nelkenöl, Geraniumöl, Salbeiöl, Pimentöl, Thymianöl, Majoranöl, Basilikumöl, Citrusöl, Gaultheriaöl oder eine/mehrere daraus isolierte bzw. synthetisch erzeugte Komponenten dieser Öle enthalten sein. Die wichtigsten Komponenten der genannten Öle sind z. B. Menthol, Carvon, Anethol, Cineol, Eugenol, Zimtaldehyd, Caryophyllen, Geraniol, Citronellol, Linalool, Salven, Thymol, Terpinen, Terpinol, Methylchavicol und Methylsalicylat. Weitere geeignete Aromen sind z. B. Menthylacetat, Vanillin, Jonone, Linalylacetat, Rhodinol und Piperiton.

Eine besonders bevorzugte Ausführungsform des Zahnreinigungsmittel enthält a) 5 - 30 Gew.-% eines Poliermittels; b) 20 - 85 Gew.-% eines Feuchthaltemittels; c) 0,3 - 5 Gew.-% eines Tensids oder Tensidgemisches; d) 0,01 - 5 Gew.-% einer wasserunlöslichen, nicht-derivatisierten Cellulose e) 0,05 - 0,5 Gew.-% eines Fluorids; f) 5 - 50 Gew.-% Wasser g) 1 - 10 Gew.-% eines C₄- und/oder C₅-haltigen Treibgases oder Treibgasgemisches und in einem Spendersystem mit manuell betätigbarem Ventil. Diese Kombination ermöglicht einen besonders guten post-foaming Effekt, eine sehr leichte Verteilbarkeit und schnelle Aromafreisetzung sowie einen besonders formbeständigen Schaum.

### Weitere übliche Inhaltsstoffe

Das erfindungsgemäße Mittel kann vorzugsweise eine Reihe weiterer Zahnpastainhaltsstoffe enthalten. Hierzu gehören u. a.:
- Vitamine, z. B. Retinol, Biotin, Tocopherol, Ascorbinsäure und deren Derivate (z. B. Ester, Salze);
- Pigmente, z. B. Titandioxid oder Zinkoxid;
- Bleichmittel;
- Farbstoffe;
- pH-Stellmittel und Puffersubstanzen, z. B. Natriumcitrat, Natriumbicarbonat oder Kalium- und Natriumphosphate,
- Natriumbenzoat;
- wundheilende und entzündungshemmende Stoffe wie z. B. Allantoin, Harnstoff, Panthenol, Azulen oder Kamillenextrakt, Acetylsalicylsäure-Derivate, Rhodanid; Wasserstoffperoxid
- Zink- und Mangansulfat

### Erfindungsgemäße Beispiele

Die Herstellung des Zahnpflegereinigungsmittels erfolgte unter Entgasung, d. h. im mäßigen Vakuum (≤ 50 mbar), bei Raumtemperatur.

### Allgemeine Vorschrift

Die Feuchthaltemittel, Kieselsäuren und ggf. weitere Poliermittel wurden zusammen mit entsalztem Wasser im Vakuum unter Rühren homogenisiert. Anschließend wurden die löslichen Salze und Farbstoffe zugegeben. Die Cellulose wurde in etwas Wasser suspendiert und in die Masse eingearbeitet. Weitere organische Bindemittel (z. B. Xanthan gum etc.) wurden in PEG dispergiert und anschließend unter Rühren in die Masse eingearbeitet. Nach Quellung der Masse wurden die Ölkomponenten und Tenside, die vorher miteinander bei 60 - 85 ° C emulgiert worden waren, zugegeben, dann die Aromastoffe und solange weitergerührt, bis eine homogene Masse mit glatter Struktur erhalten wurde.

Die Pastenmasse (90 - 99 Gew.-%) wurde zusammen mit 1 - 10 Gew.-% des Treibgases/Treibgasgemisches in das Atmos™ Dispensing System (70 oder 140 mL) der Exxel Container Inc. oder in eine Zweikammer-Aerosol-Dose unter den für solche Systeme üblichen Bedingungen abgefüllt (Abfülldruck: ca. 40 bar).

Die Mengenangaben in nachfolgenden Beispielen beziehen sich, soweit nicht anders angegeben, auf Gew.-% Aktivsubstanz der Gesamtzusammensetzung ohne Treibgas.

### Beispiel 1 (Zusammensetzung ohne Treibgas)

| | |
|---|---|
| 12,0 | Gew.-% Silica (z. B. Sident® 8) |
| 60,0 | Gew.-% Sorbitol (z. B. Neosorb® 70/70 B; 70 Gew.-% in Wasser) |
| 1,5 | Gew.-% Natriumlaurylsulfat (z. B. Texapon® K 1296) |
| 0,6 | Gew.-% Cocamidopropylbetain (z. B. Tego-Betain® BL 215; 30 Gew.-% in Wasser) |
| 0,5 | Gew.-% PEG-30-Glycerylstearat (z. B. Tagat® S) |
| 1,5 | Gew.-% Polyethylenglykol MG =1550 (z. B. Polywachs® 1550) |
| 0,75 | Gew.-% Cellulose (z. B. Arbocel® CGP 5000; 5 Gew.-% in Wasser) |
| 0,3 | Gew.-% Xanthan gum (z. B. Keltrol® F) |
| 0,2 | Gew.-% Na₂HPO₄ |
| 0,32 | Gew.-% NaF |
| 0,2 | Gew.-% Natriumsaccharinat |
| 0,49 | Gew,-% Natriumbenzoat |
| 0,1 | Gew.-% Triclosan (z. B. Irgacare® MP) |
| 0,26 | Gew.-% Farbstofflösung (z. B. Kombination aus: Brillant Blue FCF, 1 Gew.-% in Wasser und Schwarz CI 27755, 0,1 Gew.-% in Wasser) |
| 1,0 | Gew.-% Aromaöl |
| ad 100 | Gew.-% Wasser VE |
| 97 | Gew.-% Zahnpastamasse wurden mit 3 Gew.-% Butangas in das Atmos™ Dispensing System von Exxel abgefüllt. |

### Beispiel 2 (Zusammensetzung ohne Treibgas)

| | |
|---|---|
| 12,0 | Gew.-% Silica (z. B. Sident® 8) |
| 60,0 | Gew.-% Sorbitol (z. B. Neosorb® 70/70 B; 70 Gew.-% in Wasser) |
| 1,5 | Gew.-% Natriumlaurylsulfat (z. B. Texapon® K 1296) |
| 0,6 | Gew.-% Cocamidopropylbetain (z. B. Tego-Betain® BL 215; 30 Gew.-% in Wasser) |
| 0,5 | Gew.-% PEG-30-Glycerylstearat (z. B. Tagat® S) |
| 1,0 | Gew.-% Jojobaöl |
| 1,5 | Gew.-% Polyethylenglykol MG = 1550 (z. B. Polywachs® 1550) |
| 1,0 | Gew.-% Cellulose (z. B. Arbocel® CGP 5000; 5 Gew.-% in Wasser) |
| 0,55 | Gew.-% Xanthan gum (z. B. Keltrol® F) |
| 0,2 | Gew.% Na₂HPO₄ |
| 0,32 | Gew.-% NaF |
| 0,2 | Gew.-% Natriumsaccharinat |
| 0,49 | Gew,-% Natriumbenzoat |
| 0,1 | Gew.-% Triclosan (z. B. Irgacare® MP) |
| 0,26 | Gew.-% Farbstofflösung (z. B. Kombination aus: Brillant Blue FCF, 1 Gew.-% in Wasser und Schwarz CI 27755, 0,1 Gew.-% in Wasser) |
| 1,0 | Gew.-% Aromaöl |
| ad 100 | Gew.-% Wasser VE |
| 96 | Gew.-% Zahnpastamasse wurden mit 4 Gew.-% Butan in eine Zweikammer-Aerosol-dose abgefüllt. |

### Beispiel 3 (Zusammensetzung ohne Treibgas)

| | |
|---|---|
| 12,0 | Gew.-% Silica (z. B. Sident® 8) |
| 60,0 | Gew.-% Sorbitol (z. B. Neosorb® 70/70 B; 70 Gew.-% in Wasser) |
| 1,5 | Gew.-% Natriumlaurylsulfat (z. B. Texapon® K 1296) |
| 0,6 | Gew.-% Cocamidopropylbetain (z. B. Tego-Betain® BL 215; 30 Gew.-% in Wasser) |
| 0,5 | Gew.-% PEG-30-Glycerylstearat (z. B. Tagat® S) |
| 1,0 | Gew.-% Jojobaöl |
| 1,0 | Gew.-% Cellulose (z. B. Arbocel® CGP 5000; 5 Gew.-% in Wasser) |
| 0,65 | Gew.-% Xanthan gum (z. B. Cekol® 2000 H) |
| 1,5 | Gew.-% Polyethylenglykol MG = 1550 (z. B. Polywachs® 1550) |
| 0,2 | Gew.-% Na₂HPO₄ |
| 0,32 | Gew.% NaF |
| 0,2 | Gew.-% Natriumsaccharinat |
| 0,49 | Gew,-% Natriumbenzoat |
| 0,1 | Gew.-% Triclosan (z. B. Irgacare® MP) |
| 0,26 | Gew.-% Farbstofflösung (z. B. Kombination aus: Brillant Blue FCF, 1 Gew.-% in Wasser und Schwarz CI 27755, 0,1 Gew.-% in Wasser) |
| 1,0 | Gew.-% Aromaöl |
| ad 100 | Gew.-% Wasser VE |
| 98 | Gew.-% Zahnpastamasse wurden mit 2 Gew.-% eines 3:1-Gemisches (Gewichtsantei-le) aus Isopentan und Isobutan in das Atmos™ Dispensing System von Exxel abgefüllt. |

### Beispiel 4 (Zusammensetzung ohne Treibgas)

| | |
|---|---|
| 12,0 | Gew.-% Silica (z. B. Sident® 8) |
| 60,0 | Gew.-% Sorbitol (z. B. Neosorb® 70/70 B; 70 Gew.-% in Wasser) |
| 1,5 | Gew.-% Natriumlaurylsulfat (z. B. Texapon® K 1296) |
| 0,6 | Gew.-% Cocamidopropylbetain (z. B. Tego-Betain® BL 215; 30 Gew.-% in Wasser) |
| 0,5 | Gew.-% PEG-30-Glycerylstearat (z. B. Tagat® S) |
| 1,0 | Gew.-% Jojobaöl |
| 2,0 | Gew.-% Cellulose (z. B. Vitacel® L 600) |
| 0,7 | Gew.-% Xanthan gum (z. B. Cekol® 2000 H) |
| 0,03 | Gew.-% Polyvinylpyrrolidin (z. B. Luviskol®K 30) |
| 1,5 | Gew.-% Polyethylenglykol MG = 1550 (z. B. Polywachs® 1550) |
| 0,2 | Gew.-% Na₂HPO₄ |
| 0,32 | Gew.-% NaF |
| 0,2 | Gew.-% Natriumsaccharinat |
| 0,49 | Gew,-% Natriumbenzoat |
| 0,10 | Gew.-% Triclosan (z. B. Irgacare® MP) |
| 0,02 | Gew.-% Farbstoff (z. B. Sicumet Blau CI 74160) |
| 1,0 | Gew.-% Aromaöl |
| ad 100 | Gew.-% Wasser VE |
| 96 | Gew.-% Zahnpastamasse wurden mit 4 Gew.-% eines 3:1-Gemisches (Gewichtsantei-le) aus Isopentan und Isobutan in eine Zweikammer-Aerosoldose abgefüllt. |

### Anhang

1) Arbocel® CGP 5000
   Cellulose-Paste (5 Gew.-% in Wasser)
   Hersteller: J. Rettenmaier & Söhne GmbH + CO
2) Brilliant Blue® FCF
   INCI: Acid blue 9/ CI 42090
   Hersteller: Fiorio Colori S.p.A. (Pharmorgana)
3) BP-Energol® WM-6
   Paraffinöl, dickflüssig
   Hersteller: BP
4) Cekol®2000 H
   INCI: Cellulose (Na-Carboxymethylcellulose)
   Hersteller: Metsa (Nordmann Rassmann)
5) Irgacare® MP
   INCI: Triclosan
   Hersteller: Ciba
6) Keltrol® F
   INCI: Xanthan Gum
   Hersteller: Kelco
7) Laponite® DS
   Natrium-Magnesium-Lithium-Schichtsilikat
   Hersteller: Laporte
8) Luviskol® K 30
   INCI: PVP
   Hersteller: BASF
9) Neosorb® 70/70 B
   INCI: Sorbitol (70 Gew.-% in Wasser)
   Hersteller: Roquette
10) Polywachs® 1550
   Polyethylenglykol, MG = 1550
   Hersteller: RWE/DEA (Contensio, ehemals Hüls)
11) Sident® 8
   INCI: Hydrated silica
   Hersteller: Degussa-Hüls
12) Tagat® S
   INCI: PEG-30 Glyceryl Stearate
   Hersteller: Goldschmidt
13) Tego-Betain® BL 215 (30 Gew.-% in Wasser)
   INCI: Cocamidopropyl betaine
   Hersteller: Goldschmidt
14) Texapon® K 1296
   INCI: Sodium lauryl sulfate
   Hersteller: Cognis Deutschland GmbH (Henkel)
15) Vitacel® L 600
   Cellulose-Pulver
   Hersteller: J. Rettenmaier & Söhne GmbH + CO

## Patentansprüche

1. Zahnreinigungsmittel enthaltend
a) wenigstens ein Poliermittel
b) wenigstens ein Feuchthaltemittel
c) wenigstens ein Tensid
d) wenigstens eine nicht-derivatisierte, wasserunlösliche Cellulose
**dadurch gekennzeichnet, daß** es mit einem Treibgas oder Treibgasgemisch in einem Spendersystem mit manuell betätigbarem Ventil abgefüllt ist.

2. Zahnreinigungsmittel gemäß Anspruch 1, **dadurch gekennzeichnet, daß** das Treibgas wenigstens eine Komponente mit einem Siedepunkt von -5 - 35° C enthält.

3. Zahnreinigungsmittel gemäß einem der Ansprüche 1 oder 2, **dadurch gekennzeichnet, daß** es wenigstens 1 Gew.-% eines Treibgases oder eines Treibgasgemisches enthält.

4. Zahnreinigungsmittel gemäß einem der Ansprüche 1 bis 3, **dadurch gekennzeichnet, daß** das Treibgas ein C₅-Alkan enthält.

5. Zahnreinigungsmittel gemäß einem der Ansprüche 1 bis 4, **dadurch gekennzeichnet, daß** das Treibgas ein Gemisch eines C₄-Alkans und eines C₅-Alkans enthält.

6. Zahnreinigungsmittel gemäß einem der Ansprüche 1 bis 5, **dadurch gekennzeichnet, daß** wenigstens ein Poliermittel enthalten ist, das aus der Gruppe der Kieselsäuren oder Silikate gewählt ist.

7. Zahnreinigungsmittel gemäß einem der Ansprüche 1 bis 6, **dadurch gekennzeichnet, daß** wenigstens ein Feuchthaltemittel gewählt aus der Gruppe Sorbitol, Glycerin, Xylitol bzw. ein beliebiges Gemisch dieser Substanzen enthalten ist.

8. Zahnreinigungsmittel gemäß einem der Ansprüche 1 bis 7, **dadurch gekennzeichnet, daß** wenigstens ein Tensid aus der Gruppe der Aniontenside enthalten ist.

9. Zahnreinigungsmittel gemäß einem der Ansprüche 1 bis 8, **dadurch gekennzeichnet, daß** es zusätzlich wenigsten eine Öl- und/oder Wachskomponente enthält.

10. Zahnreinigungsmittel gemäß einem der Ansprüche 1 bis 9, **dadurch gekennzeichnet, daß** es zusätzlich wenigstens ein weiteres Binde- oder Verdickungsmittel enthält.

11. Zahnreinigungsmittel gemäß einem der Ansprüche 1 bis 10, **dadurch gekennzeichnet, daß** es als zusätzliche Wirkstoffe Antikarieswirkstoffe, antimikrobielle Wirkstoffe, Zahnstein-Inhibitoren, Remineralisierungswirkstoffe, Geschmacksstoffe oder eine beliebige Kombination dieser Stoffe enthält.

12. Zahnreinigungsmittel gemäß einem der Ansprüche 1 bis 11, **dadurch gekennzeichnet, daß** das Mittel ohne Treibgas bei 25°C eine Viskosität von wenigstens 10000 mPa·s aufweist.

13. Zahnreinigungsmittel gemäß einem der Ansprüche 1 bis 12, **dadurch gekennzeichnet, daß** es wenigstens
a) 5 - 30 Gew.-% eines Poliermittels
b) 20 - 85 Gew.-% eines Feuchthaltemittels
c) 0,3 - 5 Gew.-% eines Tensids oder Tensidgemisches
d) 0,01 - 5 Gew.-% einer wasserunlöslichen, nicht-derivatisierten Cellulose
e) 0,05 - 0,5 Gew.-% eines Fluorids
f) 5 - 50 Gew.-% Wasser
g) 1 - 10 Gew.-% eines C₄- und/oder C₅-haltigen Treibgases oder Treibgasgemisches
in einem Spendersystem mit manuell betätigbarem Ventil enthält.

14. Verwendung einer Zusammensetzung nach einem der Ansprüche 1 bis 13 zur Herstellung eines Zahnreinigungsmittels für die Reinigung und Pflege der Zähne und zur Prävention von Zahn- und Zahnfleischerkrankungen.

15. Verfahren zur Herstellung eines Mittels für die Zahnpflege und -reinigung, **dadurch gekennzeichnet, dass** man aus einem Spendersystem durch manuelle Betätigung eines Ventils einen nicht oder nur wenig aufgeschäumten Zahnpastastrang auf einen zur mechanischen Reinigung der Zähne geeigneten Gegenstand aufbringt, wobei der Zahnpastastrang bei 20°C und Normaldruck eine Expansionsrate von nicht mehr als 100 Vol.-% in 5 Sekunden aufweist und dann in einer Zeitspanne von 10 bis 100 Sekunden auf nicht mehr als das 6-fache seines Volumens expandiert, und man das gashaltige Zahnpsta-Mousse zur Reinigung der Zahnoberflächen, Zahnzwischenräume und der Mundhöhle verwendet.

## Claims

1. Tooth cleaning agent comprising
a) at least one polishing agent
b) at least one humectant
c) at least one surfactant
d) at least one non-derivatized, water-insoluble cellulose
**characterized in that** it is bottled with a propellant gas or propellant gas mixture in a dispensing system with manually actuatable valve.

2. Tooth cleaning agent according to Claim 1, **characterized in that** the propellant gas comprises at least one component with a boiling point of from -5 - 35°C.

3. Tooth cleaning agent according to one of Claims 1 or 2, **characterized in that** it comprises at least 1% by weight of a propellant gas or of a propellant gas mixture.

4. Tooth cleaning agent according to one of Claims 1 to 3, **characterized in that** the propellant gas comprises a C₅-alkane.

5. Tooth cleaning agent according to one of Claims 1 to 4, **characterized in that** the propellant gas comprises a mixture of a C₄-alkane and a C₅-alkane.

6. Tooth cleaning agent according to one of Claims 1 to 5, **characterized in that** at least one polishing agent is present which is chosen from the group of silicas or silicates.

7. Tooth cleaning agent according to one of Claims 1 to 6, **characterized in that** at least one humectant chosen from the group consisting of sorbitol, glycerol, xylitol or any mixture of these substances is present.

8. Tooth cleaning agent according to one of Claims 1 to 7, **characterized in that** at least one surfactant from the group of anionic surfactants is present.

9. Tooth cleaning agent according to one of Claims 1 to 8, **characterized in that** it additionally comprises at least one oil component and/or wax component.

10. Tooth cleaning agent according to one of Claims 1 to 9, **characterized in that** it additionally comprises at least one further binder or thickener.

11. Tooth cleaning agent according to one of Claims 1 to 10, **characterized in that** it comprises, as additional active ingredients, anticaries active ingredients, antimicrobial active ingredients, tartar inhibitors, remineralizing active ingredients, flavourings or any combination of these substances.

12. Tooth cleaning agent according to one of Claims 1 to 11, **characterized in that** the agent without propellant gas has a viscosity of at least 10 000 mPa·s at 25°C.

13. Tooth cleaning agent according to one of Claims 1 to 12, **characterized in that** it comprises at least
a) 5 - 30% by weight of a polishing agent
b) 20 - 85% by weight of a humectant
c) 0.3 - 5% by weight of a surfactant or surfactant mixture
d) 0.01 - 5% by weight of a water-insoluble, non-derivatized cellulose
e) 0.05 - 0.5% by weight of a fluoride
f) 5 - 50% by weight of water
g) 1 - 10% by weight of a C₄- and/or C₅-containing propellant gas or propellant gas mixture
in a dispensing system with manually actuatable valve.

14. Use of a composition according to one of Claims 1 to 13 for the preparation of a tooth cleaning agent for the cleaning and care of the teeth and for the prevention of dental and gum diseases.

15. Process for the preparation of an agent for tooth care and cleaning, **characterized in that** from a dispensing system, through manual actuation of a valve, an unfoamed or only slightly foamed toothpaste strand is applied to an object suitable for the mechanical cleaning of teeth, where the toothpaste strand has an expansion rate of not more than 100% by volume in 5 seconds at 20°C and atmospheric pressure and then expands to not more than 6 times its volume within a period from 10 to 100 seconds, and the gas-containing toothpaste mousse is used for cleaning the surfaces of the teeth, spaces between the teeth and the oral cavity.

## Revendications

1. Produit de nettoyage des dents contenant
a) au moins un agent de polissage,
b) au moins un agent de maintien de l'humidité,
c) au moins un tensioactif,
d) au moins une cellulose non dérivatisée, insoluble dans l'eau,
**caractérisé en ce qu'**il est conditionné dans un système distributeur muni d'une valve à actionnement manuel, conjointement avec un gaz propulseur ou un mélange de gaz propulseurs.

2. Produit de nettoyage des dents selon la revendication 1, **caractérisé en ce que** le gaz propulseur contient au moins un composant ayant un point d'ébullition de -5 à 35°C.

3. Produit de nettoyage des dents selon l'une des revendications 1 ou 2,
**caractérisé en ce qu'**il contient au moins 1 % en poids d'un gaz propulseur ou d'un mélange de gaz propulseurs.

4. Produit de nettoyage des dents selon l'une quelconque des revendications 1 à 3, **caractérisé en ce que** le gaz propulseur contient un alcane en C₅.

5. Produit de nettoyage des dents selon l'une quelconque des revendications 1 à 4, **caractérisé en ce que** le gaz propulseur contient un mélange d'un alcane en C₄ et d'un alcane en C₅.

6. Produit de nettoyage des dents selon l'une quelconque des revendications 1 à 5, **caractérisé en ce qu'**il contient au moins un agent de polissage, choisi dans le groupe des acides siliciques ou des silicates.

7. Produit de nettoyage des dents selon l'une quelconque des revendications 1 à 6, **caractérisé en ce qu'**il contient au moins un agent de maintien de l'humidité choisi dans le groupe constitué par le sorbitol, le glycérol, le xylitol, ou un mélange quelconque de ces substances.

8. Produit de nettoyage des dents selon l'une quelconque des revendications 1 à 7, **caractérisé en ce qu'**il contient au moins un tensioactif choisi dans le groupe des tensioactifs anioniques.

9. Produit de nettoyage des dents selon l'une quelconque des revendications 1 à 8, **caractérisé en ce qu'**il contient en outre au moins un composant huileux ou cireux.

10. Produit de nettoyage des dents selon l'une quelconque des revendications 1 à 9, **caractérisé en ce qu'**il contient en outre au moins un autre liant ou épaississant.

11. Produit de nettoyage des dents selon l'une quelconque des revendications 1 à 10, **caractérisé en ce qu'**il contient, en tant que principes actifs supplémentaires, des principes actifs anti-caries, des principes actifs antimicrobiens, des inhibiteurs de tartre, des principes actifs de reminéralisation, des agents de goût ou une combinaison quelconque de ces substances.

12. Produit de nettoyage des dents selon l'une quelconque des revendications 1 à 11, **caractérisé en ce qu'**il présente, sans gaz propulseur, à 25°C, une viscosité d'au moins 10 000 mPa.s.

13. Produit de nettoyage des dents selon l'une quelconque des revendications 1 à 12, **caractérisé en ce qu'**il contient au moins
a) 5-30 % en poids d'un agent de polissage
b) 20-85 % en poids d'un agent de maintien de l'humidité
c) 0,3-5 % en poids d'un tensioactif ou d'un mélange de tensioactifs,
d) 0,01-5 % en poids d'une cellulose non dérivatisée, insoluble dans l'eau
e) 0,05-0,5 % en poids d'un fluorure
f) 5-50 % en poids d'eau
g) 1-10 % en poids d'un gaz propulseur ou d'un mélange de gaz propulseurs qui contient des alcanes en C₄ et/ou des alcanes en C₅
dans un système distributeur muni d'une valve à actionnement manuel.

14. Utilisation d'une composition selon l'une quelconque des revendications 1 à 13, pour la préparation d'un produit de nettoyage des dents destiné au nettoyage et au soin des dents et à la prévention des affections des dents et de la gencive.

15. Procédé de préparation d'un produit destiné au nettoyage et au soin des dents, **caractérisé en ce que** l'on applique à partir d'un système distributeur, par actionnement manuel d'une valve, un cordon de pâte dentifrice non mousseux ou que faiblement mousseux, sur un objet approprié pour le nettoyage mécanique des dents, le cordon de pâte dentifrice présentant à 20°C et à la pression ordinaire un taux d'expansion qui n'est pas supérieur à 100 % en volume dans les 5 secondes, et ensuite, en une durée de 10 à 100 secondes, subit une expansion à un volume qui n'est pas supérieur à 6 fois son volume, et l'on utilise la mousse de pâte dentifrice contenant du gaz pour le nettoyage de la surface dentaire, des interstices dentaires et de l'orifice buccal.
